# EUROPEAN PATENT SPECIFICATION

(11) **EP 3 755 167 B1**
(45) Date of publication and mention of the grant of the patent: **19.10.2022**
(21) Application number: 19709126.7
(22) Date of filing: 15.02.2019
(51) Int. Cl.: A24F 42/20, A61M 15/00, A61M 15/06, A24F 42/60

(54) **DRY POWDER INHALER**
TROCKENPULVERINHALATOR
INHALATEUR DE POUDRE SÈCHE

(30) Priority: 19.02.2018 EP 18157483
(43) Date of publication of application: 30.12.2020
(73) Proprietor: Philip Morris Products S.A., 2000 Neuchâtel (CH)
(72) Inventor: ZUBER, Gerard, 2000 Neuchâtel (CH); GRANT, Christopher, 2000 Neuchâtel (CH); SANNA, Daniele, 2000 Neuchâtel (CH); SECHI, Gianluca, 1007 Lausanne (CH); WALLER, Judith, 2000 Neuchâtel (CH); MELONCELLI, Niki, 40069 Zola Predosa (IT)
(74) Representative: Parsi Mendiola, Joshua
(86) International application number: PCT/IB2019/051239
(87) International publication number: WO 2019/159123

(56) References cited:
- WO-A1-2017/109678
- WO-A1-2018/007887
- WO-A1-2018/100461
- US-A1- 2003 094 173

## Description

This disclosure relates to a dry powder inhaler that provides multi-use delivery of pharmaceutically active particles.

Dry powder inhalers are not typically suitable to provide pharmaceutically active particles to the lungs at inhalation or air flow rates that are within conventional smoking regime inhalation or air flow rates. Dry powder inhalers do not always include dry powder consumable which is easily replaceable once consumed.

It is desirable to provide a dry powder inhaler system that provides multi-use delivery of pharmaceutically active particles. It is desirable that the dry powder inhaler include a modular component capsule receptacle that may be easily replaceable once consumed. It is desirable that the inhaler facilitate delivery of pharmaceutically active particles to the consumer at conventional smoking regime inhalation or air flow rates.

WO 2017/109678 discloses a capsule containing particles including nicotine wherein a single aperture extends through the capsule. The nicotine particle capsule may be a suitable consumable to be used in a dry powder inhaler.

WO 2018/007887 discloses a nicotine particle delivery consumable article including a receptacle having a body extending from a receptacle first end to an opposing receptacle second end and defining a cavity. A capsule is disposed within the cavity. The capsule contains particles including nicotine. The receptacle includes a membrane sealing the receptacle first end and an air outlet extending through the receptacle second end and into the cavity. An air inlet, close to the receptacle first end, extends through a side wall of the body and into the cavity.

US 2003/094173 discloses an aerosolization apparatus comprising a body having an inlet, an endpiece having an outlet, the endpiece being connectable to the body to define a chamber, wherein the chamber is sized to receive a capsule containing a pharmaceutical formulation in a manner which allows the capsule to move within the chamber. A connection mechanism is provided to provide selective connection of the endpiece to the body, and a locking member prevents undesired disconnection of the endpiece from the body. When a user inhales, air enters into the chamber through the inlet so that the pharmaceutical formulation is aerosolized within the chamber and the aerosolized pharmaceutical formulation is delivered to the user through the outlet. In another version, the endpiece and the body are hinged together.

WO 2018/100461 discloses an inhaler article including a body extending along a longitudinal axis from a mouthpiece end to a distal end and a capsule cavity defined within the body. A mouthpiece air channel extends from the capsule cavity to the mouthpiece end. An end cap is disposed within the distal end and extends to the capsule cavity. The end cap extends from an end cap distal end to an end cap inner end. The end cap includes an air channel extending from the end cap distal end to the end cap inner end. The air channel is non-parallel with the longitudinal axis

This disclosure is directed to a reusable dry powder inhaler suitable for providing pharmaceutically active particles, preferably including nicotine particles, that includes a mouthpiece portion removably coupled to a distal end portion. The mouthpiece portion includes a capsule cavity and a mouthpiece air channel extending from the mouthpiece end to the capsule cavity. The distal end portion includes an airflow element disposed within the distal end portion and extending a longitudinal length to the capsule cavity. The airflow element includes an airflow channel extending the longitudinal length of the airflow element and is non-parallel with the longitudinal axis. The airflow channel directs inlet air into the capsule cavity and a resealable membrane is configured to reseal when a piercing element is withdrawn from the resealable membrane.

The dry powder inhaler may include an inhaler body extending from a mouthpiece end to a distal end and include a mouthpiece portion removably coupled to a distal end portion. The mouthpiece portion extends between the mouthpiece end and a first mating end. The distal end portion extends between a second mating end and the distal end. The mouthpiece portion includes a capsule cavity defined within the first mating end and a mouthpiece air channel extending from the mouthpiece end to the capsule cavity.

The distal end portion may include a piercing element coupled to the distal end portion and an airflow element disposed within the distal end portion and extending a longitudinal length to the capsule cavity. The piercing element is configured to move between a relaxed position and a piercing position. The piercing element extends through the airflow element and resealable membrane within the airflow element and into the capsule cavity in the piercing position. The resealable membrane is configured to reseal when the piercing element moves from the piercing position to the relaxed position.

The airflow element includes one or more airflow channels that produce swirling air flow to induce rotation of the capsule contained within the capsule cavity. Rotation of the capsule with the swirling air flow may cause the capsule to release pharmaceutically active particles (once pierced) into the airflow. The release may provide a fractional amount of pharmaceutically active particles with each air flow "puff" by the consumer. Each fractional amount of dry powder released may be substantially equal or equivalent to each other. The capsule may be considered a multiple-use element. The capsule may be emptied (or depleted) of dry powder with about 8 to 25 inhalations or "puffs", for example.

Preferably the resealable membrane occludes a linear piercing channel that extends the length of the airflow element. The resealable membrane may prevent air flow into the capsule cavity via the linear piercing element.

The capsule may be a modular component of the multiple-use dry powder inhaler. The capsule may be easily replaceable within the multiple-use inhaler. Once consumed, the capsule may be removed from the multiple-use dry powder inhaler and discarded.

Advantageously, the dry powder inhaler described herein provides a re-usable modular component approach when combined with a consumable capsule. The airflow element within the distal end portion of the inhaler ensures that swirling airflow enters the capsule cavity. The airflow element may be reliably formed with a molding process and easily assembled into the inhaler. This air flow management ensures that the capsule rotates during inhalation and consumption. This rotation may suspend and aerosolize the pharmaceutically active particles in the inhalation air moving through the dry powder inhaler. The pharmaceutically active particles may be delivered with the dry powder inhaler at inhalation or air flow rates that are within conventional smoking regime inhalation or air flow rates.

The dry powder inhaler described herein may provide dry powder to the lungs at inhalation or air flow rates that are within conventional smoking regime inhalation or air flow rates. A consumer may take a plurality of inhalations or "puffs" where each "puff" delivers a fractional amount of dry powder contained within a capsule contained within the capsule cavity. This dry powder inhaler may mimic the ritual of conventional smoking. This dry powder inhaler may be simple to manufacture and convenient to use by a consumer.

Air flow management through the capsule cavity may cause the capsule to rotate during inhalation and consumption. The capsule contains pharmaceutically active particles in a dry powder form. This dry powder may optionally include particles comprising flavour, also referred to as "flavour particles.

A preferred pharmaceutically active particle is nicotine particle. Nicotine particles comprise nicotine and may be referred to as "nicotine powder" or "nicotine particles".

Rotation of the pierced capsule may suspend and aerosolize the pharmaceutically active particles released from the pierced capsule into the inhalation air moving through the dry powder inhaler. The flavour particles may be larger than the pharmaceutically active particles and may assist in transporting the pharmaceutically active particles into the lungs of the user while the flavour particles preferentially remain in the mouth or buccal cavity of the user. The pharmaceutically active particles and optional flavor particles may be delivered with the dry powder inhaler article at inhalation or air flow rates that are within conventional smoking regime inhalation or air flow rates.

The term "pharmaceutically active" particle refers to particles that alters one or more chemical or physiological functions of a cell, tissue, organ, or organism. A pharmaceutically active component of a particle may be, for example, a systemic or local drug, a peptide or DNA based drug, an anti-inflammatory agent, a bronchodilating agent, an antiviral agent, an antibiotic agent, an immunostimulatory agent, an immunosupressive agent, an anesthetic agent, an anticancer agent, a vitamin, a hormone, an antiepileptic agent, an antifungal agent, an antioxidant, an antidiabetic agent, a muscle relaxant, and anti-HIV agent, a stimulant, a cough suppressant, a pain controller, a smoking cessation agent or an alcohol abuse agent. Preferably the pharmaceutically active component of a particle is nicotine.

The term "nicotine" refers to nicotine and nicotine derivatives such as free-base nicotine, nicotine salts and the like.

The term "flavourant" or "flavour" refers to organoleptic compounds, compositions, or materials that alter and are intended to alter the taste or aroma characteristics of nicotine during consumption or inhalation thereof. The term "flavourant" or "flavour" preferably refers to compounds disclosed in the Flavor & Extract Manufacturers Association (FEMA) Flavor Ingredient Library and in particular in the GRAS Flavoring Substances publications 3 to 27, for example, see Hall, R.L. & Oser, B.L., Food Technology, February 1965 pg 151-197, and in the GRAS flavoring substances 27 S.M. Cohen et al., Food Technology Aug. 2015 pg. 40-59, and intervening GRAS Flavoring Substances publications 4 to 26. For the purpose of this disclosure, nicotine is not considered as a flavourant or flavour.

The size of a particle, stated herein, preferably refers to the aerodynamic diameter of the particle. The aerodynamic diameter of the particles is preferably measured with a cascade impactor.

The dry powder inhaler described herein may be combined with one or more modular pharmaceutically active particle delivery consumables (capsules) to deliver pharmaceutically active particles to a consumer. A plurality of these modular pharmaceutically active particle delivery consumables (similar or different formulation or flavors) may be combined with a dry powder inhaler to form a kit.

The dry powder inhaler described herein may be combined with a piercing element or piercing device to deliver the pharmaceutically active particles to a consumer. The piercing element or piercing device may be separated from or not form a portion of the dry powder inhaler article. A plurality of these dry powder inhaler articles may be combined with a piercing element or piercing device to form a kit.

Alternatively, the dry powder inhaler described herein may include a piercing element or piercing device coupled to the distal end portion.

The dry powder inhaler includes a mouthpiece portion removably coupled to a distal end portion. The mouthpiece portion includes a capsule cavity and a mouthpiece air channel extending from the mouthpiece end to the capsule cavity. The distal end portion includes an airflow element disposed therein. The airflow element includes an airflow channel (or two or more airflow channels) extending the longitudinal length of the airflow element and is non-parallel with the longitudinal axis. The airflow channel directs inlet air into the capsule cavity. The airflow element includes a resealable membrane configured to reseal when a piercing element is withdrawn from the resealable membrane.

The dry powder inhaler body extends from a mouthpiece end to a distal end. The inhaler body includes a mouthpiece portion removably coupled to a distal end portion. The mouthpiece portion extending between the mouthpiece end and a first mating end. The distal end portion extending between a second mating end and the distal end.

The mouthpiece portion includes a capsule cavity defined within the first mating end, and a mouthpiece air channel extending from the mouthpiece end to the capsule cavity.

The distal end portion includes an airflow element disposed within the distal end portion and extends a longitudinal length to the capsule cavity. The airflow element includes an airflow channel extending the longitudinal length of the airflow element and is non-parallel with the longitudinal axis. The airflow channel directs inlet air into the capsule cavity. The airflow element includes a resealable membrane configured to reseal when a piercing element is withdrawn from the resealable membrane.

In some embodiments, the distal end portion further includes a piercing element coupled to the distal end portion. The piercing element may be coupled to the distal end portion and adjacent to the distal end. The piercing element may be configured to move between a relaxed position and a piercing position. The piercing element extending through the resealable membrane and into the capsule cavity in the piercing position. The piercing element being spaced apart from the resealable membrane in the relaxed position.

In these embodiments, the distal end portion may surround a portion of the piercing element. The piercing element may move along a longitudinal axis between a piercing (activated) position and a relaxed position. A biasing element may maintain the piercing element in the relaxed position. The biasing element may be a spring member. The biasing element may compress when the piercing element is pressed into the piercing position. Releasing the piercing element allows the biasing element to force the piercing element back to the relaxed position.

The piercing element may be a rigid element capable of piercing the resealable membrane and a capsule contained within the capsule cavity. The piercing element may be a metal element such as a needle.

The one or more airflow channels through the airflow element initiates "swirling" air flow though the capsule cavity. The swirling air flow may cause a capsule contained within the capsule cavity to rotate and release pharmaceutically active particles (once pierced) into the airflow through the dry powder inhaler. Preferably there may be more than one airflow channel through the airflow element. The airflow element extends from a first lateral (orthogonal to the longitudinal axis) face to an opposing second lateral face a longitudinal length defining the distance between the first lateral face to an opposing second lateral face. The airflow channel preferably extends the total longitudinal length of the airflow element. The first lateral face may define the upstream boundary of the capsule cavity when the distal end portion is coupled to the mouthpiece portion. The airflow channel may extend from the first lateral face to the second lateral face and define a curved or arcuate path.

The airflow channel may be continuously non-parallel with the longitudinal axis of the inhaler along an entire length of the airflow channel. The airflow channel may be parallel along a portion of the length of the airflow channel and non-parallel along a remaining portion of the length of the airflow channel. The airflow channel may be parallel for a first portion or upstream portion of the airflow channel and be non-parallel for a second portion or downstream portion of the airflow channel exiting into the capsule cavity. The second portion may define a about 50% or less, or from about 5% to about 50%, or from about 10% to about 30% of the total airflow channel length.

The airflow channel may be a channel element defined along an outer surface of the airflow element and extending along a length of the airflow element. The airflow element may define three sides of the airflow channel. The airflow element may define a bottom surface and opposing depth sides that define a depth of the airflow channel. The airflow element may be inserted into the distal end portion of the inhaler body and optionally form a portion of the distal end of the inhaler body. The inhaler body may surround at least about 75%, or at least about 85% or at least about 90% or 100% of the length of the airflow element. The distal end portion of the inhaler body may contain and hold the airflow element in place within the distal end portion of the inhaler body.

The airflow element may be inserted into the distal end portion of the inhaler body and may be fixed to the inhaler body by friction fit, mechanical fit, or an adhesive, for example. A distal end portion of the inhaler body may cooperate with the airflow element airflow channel to enclose the airflow channel or form the remaining top surface of the airflow channel. The top surface may oppose the bottom surface defined by the airflow element. The top surface and bottom surface may be parallel to each other. The opposing depth sides may be parallel to each other. The opposing top surface and bottom surface may be orthogonal to the opposing depth sides.

The airflow channel may extend a distance along an arc that is co-axial with the longitudinal axis. The airflow channel may be curved with respect the longitudinal axis of the inhaler. The airflow channel may rotate around the circumference of the airflow element as a function of a location along the airflow element longitudinal length. The airflow channel may rotate around about 5% to about 100%, or about 25% to about 50% of the circumference of the airflow element. The airflow channel may rotate around the circumference of the airflow element an arc length (distance when viewing the airflow element from the first lateral face) having a central angle (that may be coincident with the longitudinal axis of the inhaler body) in a range from about or from about 5 degrees to about 360 degrees, or about 45 degrees to about 180 degrees, or from about 45 degrees to about 135 degrees.

The airflow channel may enter the capsule cavity at an angle relative to the longitudinal axis. The airflow channel may enter the capsule cavity at an angle in a range from about 5 degrees to about 89 degrees, or about 45 degrees to about 89 degrees, or about 60 degrees to about 89 degrees, or about 70 degrees to about 88 degrees. The airflow channel may have a first portion parallel with the longitudinal axis and a second portion exiting into the capsule cavity at an angle relative to the longitudinal axis as described above.

The airflow channel may include one, or two, or two or more air channels formed into the airflow element. The airflow channel may include at least two, or three or more air channels formed into the airflow element. The airflow channels may be located symmetrically about the airflow element. The airflow channels may oppose each other about the airflow element along the airflow element length. The airflow channels may be equally spaced apart from each other about the airflow element along the airflow element length. The one or more airflow channels may have a helical shape (forming a portion of a spiral). The helical air channels may be symmetrically disposed along the airflow element length and preferably oppose each other along the airflow element length. The airflow channels may each extend a distance along an arc that are each co-axial with the longitudinal axis. The inhaler body may form the top surface for each airflow channel.

The airflow element and airflow channel defined thereon may be precisely designed and manufactured to impart the desired air flow pattern through the capsule cavity of the inhaler. This distal end portion and airflow element may form a separate piece assembly that may provide for a simple and reliable manufacture and performance of the dry powder inhaler.

The airflow element may have a longitudinal length in a range from about 3 mm to about 12 mm, or from about 4 mm to about 10 mm, or from about 5 mm to about 9 mm, or about 7 mm. The airflow element may have an outer diameter sufficient to form a close or friction fit with the inner diameter of the distal end portion. The airflow element may have an outer diameter in a range from about 5 mm to about 10 mm, or from about 6 mm to about 9 mm, or about 6.5 mm to about 8.5 mm, or about 7.5 mm.

The airflow element may include a linear piercing channel extending through the length of the airflow element. The linear piercing channel may extend along a central axis of the airflow element. The linear piercing channel may be co-axial or coincident with the longitudinal axis of the inhaler body. The linear piercing channel may be sized to allow a piercing element to pass through the linear piercing channel. The linear piercing channel may have a diameter in a range from about 0.5 mm to about 2 mm.

The airflow element may include a resealable element disposed on or within or occluding the linear piercing channel. The linear piercing channel includes a first end forming a portion of the first lateral face and an opposing second end forming a portion of the second lateral face. The resealable element may be disposed on or within the first lateral face or second lateral face. The resealable element may form a portion of the first lateral face or second lateral face.

The resealable membrane may seal the linear piercing channel. The resealable membrane may form a hermetic or airtight seal or barrier along the linear piercing channel. The linear piercing channel may be formed of a pierce-able material. A piercing element may pass through the resealable membrane and puncture the capsule within the capsule cavity. The resealable membrane may reseal once the piercing element is retracted or removed from the resealable membrane.

The resealable membrane is configured to close a void created in the resealable membrane created by a piercing element such as a needle. The resealable membrane may be pierced and seal or close the void a plurality of times, such as at least about 3 three times, or at least about 5 times, or at least about 10 times or at least about 20 times. The void is created through the thickness of the resealable membrane. The resealable membrane may have a thickness in a range from about 0.1 to about 5 millimetres, or from about 0.5 to about 2 millimetres. The resealable membrane may be formed of any resilient material. Resealable membranes may include a septum-like element. Resealable membranes may be formed of elastic material such as rubber, silicone, metal foil co-laminated with a polymer, or latex and the like.

The capsule cavity may define a cylindrical space configured to contain a capsule (that may have an obround shape). The capsule cavity may have a length of about at least 110% to less than about 200% of a length of the capsule contained therein. The capsule cavity may have a substantially uniform or uniform diameter along the length of the capsule cavity. The capsule cavity may have a substantially cylindrical or cylindrical cross-section along the length of the capsule cavity. The capsule cavity may have a uniform diameter along the length of the capsule cavity that is from about 101% to about 125%, or from about 105% to about 110% of the outer diameter of the capsule contained within the capsule cavity. The configuration of the capsule cavity may allow the capsule to rotate with stability within the capsule cavity. The longitudinal axis of the capsule may rotates with stability about the longitudinal axis of the inhaler body during inhalation. The length of the capsule cavity may form an airtight barrier.

The capsule may contain pharmaceutically active particles. The capsule cavity may have a shape similar to the shape of the capsule. The capsule cavity may have a circular cross-sectional shape and a first diameter and the capsule may have a second diameter being less than the first diameter. The second diameter may be in a range from about 80% to about 99% of the first diameter, or the second diameter may be in a range from about 90% to about 98% of the first diameter. The capsule cavity may have a length of about 20 mm and an inner diameter of about 6.6 mm when containing a capsule size 3 flat. The cavity may have a length of about 24 mm and an inner diameter of about 7.7 mm when containing a capsule size 1 flat.

The capsule rotates about its longitudinal or central axis when air flows through the inhaler. The capsule may be formed of an airtight material that may be pierced or punctured by a piercing element that may be separate or combined with the inhaler. Preferably, the piercing element forms a single hole or aperture in the capsule. The capsule preferably has an obround shape. This single, singular, or only one, hole or aperture in the capsule is preferably located on a hemispherical end of the capsule. Preferably this single, singular, or only one, hole or aperture in the capsule is preferably located on a hemispherical end of the capsule at a location that is coincident with the axis of rotation of the capsule during use.

The capsule may formed of a metallic or polymeric material that serves to keep contaminates out of the capsule but be pierced or punctured by a piercing element prior to consumption of the pharmaceutical active particles within the capsule. The capsule may be formed of a polymer material. The polymer material may be hydroxypropylmethylcellulose (HPMC). The capsule may be a size 1 to size 4 capsule, or a size 3 capsule.

A consumer may access the inhaler capsule cavity to insert the capsule consumable into the inhaler capsule cavity or replace a capsule consumable with a full or un-used capsule consumable into the inhaler capsule cavity.

The dry powder inhaler includes a mouthpiece portion removably coupled to a distal end portion. The mouthpiece portion has a first mating end that fits or mates with a second mating end of the distal portion. The first mating end couples to the second mating end. The first mating portion may snap-fit to the second mating end. The first mating portion may screw onto or be in threaded engagement with the second mating end. The first mating portion may magnetically couple with the second mating end. The first mating portion may have a friction fit with the second mating end. The first mating portion may form an air tight fit or connection with the second mating end.

The capsule may be configured to rotate about its' longitudinal or central axis when inhalation air flows through the dry powder inhaler (from the distal end air inlet through the receptacle to the mouthpiece air outlet). The capsule may be formed of an airtight material that may be pierced or punctured by the piercing element of the inhaler system.

The capsule contains a dry powder comprising pharmaceutically active particles and optionally flavour particles. The capsule may contain a predetermined amount of dry powder. The capsule may contain enough dry powder to provide at least 2 inhalations or "puffs", or at least about 5 inhalations or "puffs", or at least about 10 inhalations or "puffs". The capsule may contain enough dry powder to provide from about 5 to about 35 inhalations or "puffs", or from about 8 to about 25 inhalations or "puffs". Each inhalation or "puff" releases an approximate or substantially equal or equivalent amount of dry powder into the inhalation air stream.

The capsule may contain a dry powder about 50% to about 95% by weight pharmaceutically active particles and from 50% to 5% by weight flavour particles, or from 70% to about 90% by weight pharmaceutically active particles and from 30% to 10% by weight flavour particles. The capsule may contain from 30 mg to 70 mg of dry powder, or from 40 mg to 60 mg of dry powder.

Preferably, the capsule contains nicotine particles and flavour particles. The capsule may contain a dry powder about 50% to about 95% by weight nicotine particles and from 50% to 5% by weight flavour particles, or from 70% to about 90% by weight nicotine particles and from 30% to 10% by weight flavour particles. The capsule may contain from 30 mg to 70 mg of dry powder, or from 40 mg to 60 mg of dry powder. The nicotine particles may contain from about 1% to about 10% effective nicotine, or from about 3% to about 7% effective nicotine, or about 5% effective nicotine.

When flavour particles are blended or combined with the pharmaceutically active particles within the capsule, the flavour particles are present in an amount that provides the desired flavour to each inhalation or "puff" delivered to the user.

The pharmaceutically active particles may have any useful size distribution for inhalation delivery preferentially into the lungs of a user. The capsule may include other particles than the pharmaceutically active particles. The pharmaceutically active particles and the other particles form a powder system.

The powder system may have at least about 40% or at least about 60%, or at least about 80%, by weight of the powder system comprised in pharmaceutically active particles having a particle size of about 10 micrometres or less, or 5 micrometers or less, or in a range from about 1 micrometer to about 3 micrometres.

The powder system may have at least about 40% or at least about 60%, or at least about 80%, by weight of the powder system comprised in nicotine particles having a particle size of about 10 micrometres or less, or 5 micrometers or less, or in a range from about 1 micrometer to about 3 micrometres.

The pharmaceutically active particles may have a mass median aerodynamic diameter of about 5 micrometres or less, or in a range from about 0.5 micrometres to about 4 micrometres, or in a range from about 1 micrometres to about 3 micrometres or in a range from about 1.5 micrometres to about 2.5 micrometres. The mass median aerodynamic diameter is preferably measured with a cascade impactor.

The particles comprising nicotine may have a mass median aerodynamic diameter of about 5 micrometres or less, or in a range from about 0.5 micrometres to about 4 micrometres, or in a range from about 1 micrometres to about 3 micrometres or in a range from about 1.5 micrometres to about 2.5 micrometres. The mass median aerodynamic diameter is preferably measured with a cascade impactor.

The particles comprising flavour may have a mass median aerodynamic diameter of about 20 micrometres or greater, or about 50 micrometres or greater, or in a range from about 50 to about 200 micrometres, or from about 50 to about 150 micrometres. The mass median aerodynamic diameter is preferably measured with a cascade impactor.

The dry powder may have a mean diameter of about 60 micrometres or less, or in a range from about 1 micrometres to about 40 micrometres, or in a range from about 1.5 micrometres to about 25 micrometres. The mean diameter refers to the mean diameter per mass and is preferably measured by laser diffraction, laser diffusion or an electronic microscope.

Preferably the pharmaceutically active particle are nicotine particles. Nicotine in the powder system or nicotine particles is preferably a pharmaceutically acceptable free-base nicotine, or nicotine salt or nicotine salt hydrate. Useful nicotine salts or nicotine salt hydrates include nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. The compound combining with nicotine to form the salt or salt hydrate may be chosen based on its expected pharmacological effect.

The nicotine particles preferably include an amino acid. Preferably the amino acid is leucine such as, L-leucine. Providing an amino acid such as L-leucine with the particles comprising nicotine, may reduce adhesion forces of the particles comprising nicotine and may reduce attraction between nicotine particles and thus reduce agglomeration of nicotine particles.

Similarly, adhesion forces to particles comprising flavour is also reduced thus agglomeration of pharmaceutically active particles with flavour particles is also reduced. The powder system described herein thus may be a free-flowing material and possess a stable relative particle size of each powder component even when the pharmaceutically active particles and the flavour particles are combined.

The powder system may include flavour particles. The flavour particles may have any useful size distribution for inhalation delivery selectively into the mouth or buccal cavity of a user.

The powder system may have at least about 40%, or at least about 60%, or at least about 80%, by weight of the flavour particles of the powder system comprised in particles having a particle size of about 20 micrometres or greater. The powder system may have at least about 40% or at least about 60%, or at least about 80%, by weight of the flavour particles of the powder system comprised in particles having a particle size of about 50 micrometres or greater. The powder system may have at least about 40% or at least about 60%, or at least about 80%, by weight of the flavour particles of the powder system comprised in particles having a particle size in a range from about 50 micrometer to about 150 micrometres.

Flavourants or flavours may be provided as a solid flavour (at room temperature of about 22 degrees centigrade and one atmosphere pressure) and may include flavour formulations, flavour-containing materials and flavour precursors. The flavourant may include one or more natural flavourants, one or more synthetic flavourants, or a combination of natural and synthetic flavourants. Flavourants as described herein are organoleptic compounds, compositions, or materials that are selected and utilized to alter or are intended to alter the taste or aroma characteristics of the pharmaceutically active component during consumption or inhalation thereof.

Flavourants or flavours refer to a variety of flavour materials of natural or synthetic origin. They include single compounds and mixtures. Preferably the flavour or flavourant has flavour properties that enhance the experience of the pharmaceutically active component (preferably nicotine) during consumption. Preferably, the flavour is chosen to provide an experience similar to that resulting from smoking a combustible smoking article. For example, the flavour or flavourant may enhance flavour properties such as mouth fullness and complexity. Complexity is generally known as the overall balance of the flavour being richer without dominating single sensory attributes. Mouth fullness is described as perception of richness and volume in the mouth and throat of the consumer.

Suitable flavours include, but are not limited to, any natural or synthetic flavour, such as tobacco, smoke, menthol, mint (such as peppermint and spearmint), chocolate, licorice, citrus and other fruit flavours, gamma octalactone, vanillin, ethyl vanillin, breath freshener flavours, spice flavours such as cinnamon, methyl salicylate, linalool, bergamot oil, geranium oil, lemon oil, and ginger oil, and the like. Further suitable flavours include, horseradish oil, garlic extract, onion oil, black pepper, cayenne pepper, ginger oil, thyme oil, cinnamon bark oil, turmeric, fenugreek, cardamom, rosemary extract, grapefruit oil, andrographis extract carvacrol, thymol, monomenthyl succinate, N-(2-hydroxyethyl)-2,3-dimethyl-2-isopropyl butanamide, and the like.

Other suitable flavours may include flavour compounds selected from the group consisting of an acid, an alcohol, an ester, an aldehyde, a ketone, a pyrazine, combinations or blends thereof and the like. Suitable flavour compounds may be selected, for example, from the group consisting of phenylacetic acid, solanone, megastigmatrienone, 2-heptanone, benzylalcohol, cis-3-hexenyl acetate, valeric acid, valeric aldehyde, ester, terpene, sesquiterpene, nootkatone, maltol, damascenone, pyrazine, lactone, anethole, iso-s valeric acid, combinations thereof, and the like.

Further specific examples of flavours may be found in the current literature and are well-known to the person skilled in the art of flavouring, i.e. of imparting an odor or taste to a product.

The flavourant may be a high potency flavourant and may be used and detected at levels that would result in less than 200 parts per million in inhalation air flow. Examples of such flavourants are key tobacco aroma compounds such as beta-damascenone, 2-ethyl-3,5-dimethylpyrazine, phenylacetaldehyde, guaiacol, and furaneol. Other flavourants may only be sensed by humans at higher concentration levels. These flavourants, which are referred to herein as the lower potency flavourants, are typically used at levels that results in orders of magnitude higher amounts of flavourant released into the inhalation air. Suitable lower potency flavourants include, but are not limited to, natural or synthetic menthol, peppermint, spearmint, coffee, tea, spices (such as cinnamon, clove and ginger), cocoa, vanilla, fruit flavours, chocolate, eucalyptus, geranium, eugenol and linalool.

The particles comprising flavour may include a compound to reduce adhesion forces or surface energy and resulting agglomeration. The flavour particle may be surface modified with an adhesion reducing compound to form a coated flavour particle. One preferred adhesion reducing compound is magnesium stearate. Providing an adhesion reducing compound such as magnesium stearate with the flavour particle, especially coating the flavour particle, reduces adhesion forces of the particles comprising flavour and may reduce attraction between flavour particles and thus reduce agglomeration of flavour particles. Thus, agglomeration of flavour particles with nicotine particles is also reduced. The powder system described herein thus may possess a stable relative particle size of the particles comprising nicotine and the particles comprising flavour even when the nicotine particles and the flavour particles are combined. The powder system preferably is free flowing.

Conventional formulations for dry powder inhalation typically contain carrier particles that serve to increase the fluidization of the active particles since the active particles may be too small to be influenced by simple airflow through an inhaler. These powder systems typically require carrier particles. These carrier particles may be a saccharide such as lactose or mannitol that have a particle size greater than about 50 micrometres. The carrier particles may be utilized to improve dose uniformity by acting as a diluent or bulking agent in a formulation. These conventional formulations typically require high speed inhalation airflows and deglomeration elements and sieve elements to achieve a particle size that will enter the pulmonary system. Inhalation airflow boosting elements, deglomeration elements, and sieve elements add complexity and cost of the dry powder inhaler.

The powder system utilized with the dry powder inhaler of the invention may be carrier-free or substantially carrier-free. Being carrier-free or substantially carrier-free may allow the dry powder and to be inhaled and the pharmaceutically active particles be delivered to the user's lungs at inhalation or airflow rates that are similar to typical smoking regime inhalation or airflow rates. Preferably any carrier-like particles are limited to the flavour particles or flavour component of the dry power system.

The dry powder system may be combined in a single capsule. As described above, the dry powder system may each have reduced adhesion forces that result in a stable powder formulation where the particle size of each component does not substantially change when combined.

The inhaler described herein are less complex and have a simplified storage and airflow path as compared to conventional dry powder inhalers. Advantageously, rotation of the capsule within the inhaler system aerosolizes the dry powder system and may assist in maintaining a free-flowing powder. Thus, this dry powder inhaler does not require the typical high inhalation rates of conventional inhalers to deliver the pharmaceutically active particles described above deep into the lungs.

The inhaler system may use a flow rate of less than about 5 L/min or less than about 3 L/min or less than about 2 L/min or about 1.6 L/min. Preferably, the flow rate is in a range from about 1 L/min to about 3 L/min or from about 1.5 L/min to about 2.5 L/min. Preferably, the inhalation rate or flow rate is similar to that of Health Canada smoking regime, that is, about 1.6 L/min.

The inhaler may be used by a consumer like smoking a conventional cigarette or vaping an electronic cigarette. Such smoking or vaping is characterized by two steps: a first step during which a small volume containing the full amount of nicotine desired by the consumer is drawn into the mouth cavity, followed by a second step during which this small volume comprising the aerosol comprising the desired amount of nicotine is further diluted by fresh air and drawn deeper into the lungs. Both steps are controlled by the consumer. During the first inhalation step the consumer may determine the amount of nicotine to be inhaled. During the second step, the consumer may determine the volume for diluting the first volume to be drawn deeper into the lungs, maximizing the concentration of active agent delivered to the airway epithelial surface. This smoking mechanism is sometimes called "puff-inhale-exhale".

The dry powder utilized with the dry powder inhaler of the invention may eliminate or substantially reduce any exhalation of pharmaceutically active particles during the "exhale" phase. Preferably nearly all, or at least about 99% or at least about 95% or at least 90% of the pharmaceutically active particle has a particle size that is delivered to the lungs but are not small enough to be exhaled by tidal breathing. This pharmaceutically active particle size may be in a range from about 0.75 micrometers to about 5 micrometers, or from 0.8 micrometers to about 3 micrometers, or from 0.8 micrometers to about 2 micrometers.

All scientific and technical terms used herein have meanings commonly used in the art unless otherwise specified. The definitions provided herein are to facilitate understanding of certain terms used frequently herein.

The terms "upstream" and "downstream" refer to relative positions of elements of the inhaler described in relation to the direction of inhalation air flow as it is drawn through the body of the inhaler from a distal end portion to the mouthpiece portion.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open-ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure, including the claims.

The schematic drawings are not necessarily to scale and are presented for purposes of illustration and not limitation. The drawings depict one or more aspects described in this disclosure. However, it will be understood that other aspects not depicted in the drawing fall within the scope of the appended claims.
**FIG.** 1 is a schematic diagram of an illustrative dry powder inhaler according to the invention.
**FIG.** 2 is an exploded schematic view of the dry powder inhaler of **FIG.** 1 where the mouthpiece portion is uncoupled from the distal end portion.
**FIG. 3A** and **FIG. 3B** are perspective views of an illustrative airflow element.
**FIG. 4A** and **FIG. 4B** are perspective views of another illustrative airflow element.
**FIG. 5** is a cross-sectional schematic diagram of another illustrative airflow element.
**FIG. 6** is cross-sectional schematic diagram of another illustrative airflow element.
**FIG. 7** is a cross-sectional view of another illustrative dry powder inhaler according to the invention.

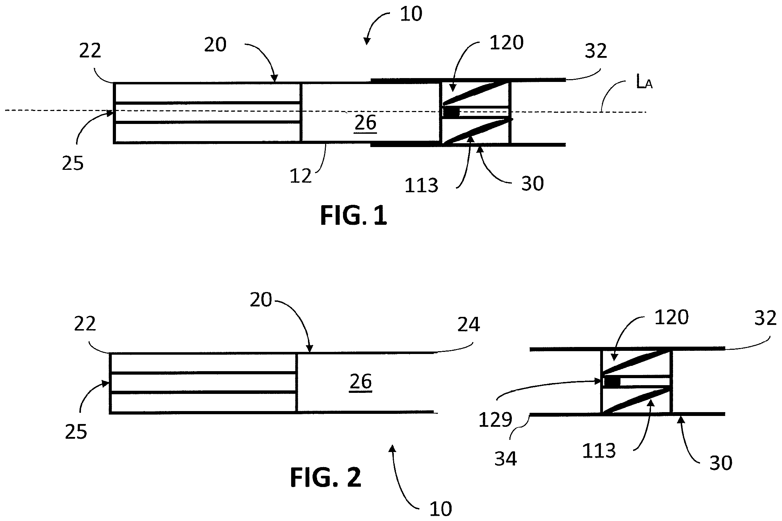

**FIG. 1** is a schematic diagram of an illustrative dry powder inhaler **10** according to the invention. **FIG 2** is a schematic diagram of the illustrative inhaler system **10** of **FIG. 1** with the distal end portion **30** un-coupled from the mouthpiece portion **20.** Referring to **FIG. 1**, a dry powder inhaler **10** includes an inhaler body **12** extending from a mouthpiece end **22** to a distal end **32.** The inhaler body **12** includes a mouthpiece portion **20** removably coupled to a distal end portion **30.** The mouthpiece portion **20** extends between the mouthpiece end **22** and a first mating end **24.** The distal end portion **30** extends between a second mating end **34** and the distal end **32.**

The mouthpiece portion **20** includes a capsule cavity **26** defined within the first mating end **24** and a mouthpiece air channel **25** extending from the mouthpiece end **22** to the capsule cavity **26.**

The distal end portion **30** includes an airflow element **120** disposed within the distal end portion **30** and extending a longitudinal length to the capsule cavity **26.** The airflow element **120** includes an airflow channel **113** extending the longitudinal length of the airflow element **120** and is non-parallel with the longitudinal axis **L_{A}**, the airflow channel **113** directs inlet air into the capsule cavity **26.** The airflow element **120** includes a resealable membrane **129** configured to reseal when a piercing element is withdrawn from the resealable membrane **129.**

**FIG 2** is a schematic diagram of the illustrative dry powder inhaler system **10** of **FIG. 1** with the distal end portion **30** un-coupled from the mouthpiece portion **20.** The distal end portion **30** may be coupled and un-coupled from the mouthpiece portion **20** with a suitable coupling means, such as, a threaded connection, a quick-connect coupling, magnetic coupling, cantilever coupling, or friction-fit coupling, for example. The first mating end **24** is complementary with the second mating end **34** and may form a generally air-tight connection.

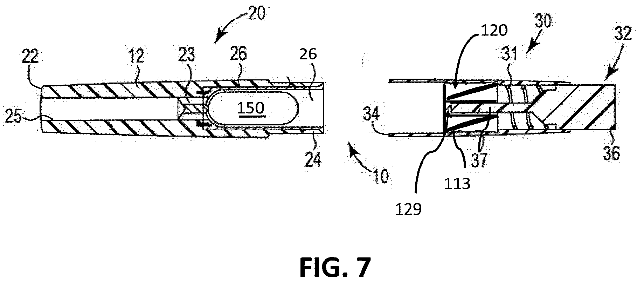

This reusable dry powder inhaler **10** may be utilized by placing a capsule **(150** see **FIG. 7**) into the capsule cavity **26** then mating the mouthpiece portion **20** to the distal end portion **30.** Then the capsule may be pierced by inserting a piercing element through the airflow element **120** resealable membrane **129** and into the capsule in the capsule cavity **26.** Then removing the piercing element from the capsule and resealable membrane **129.** A user then draws air through the inhaler **10** such that the airflow element **120** airflow channels **113** produce swirling air flow to induce rotation of a capsule contained within the capsule cavity **26** and release solid particles or dry powder from the capsule to form a depleted capsule.

The depleted capsule may then be replaced with a fresh capsule by uncoupling the mouthpiece portion **20** from the distal end portion **30** and removing the depleted capsule from the capsule cavity. A fresh or new capsule may then be placed in the capsule cavity **26** and the process repeats itself.

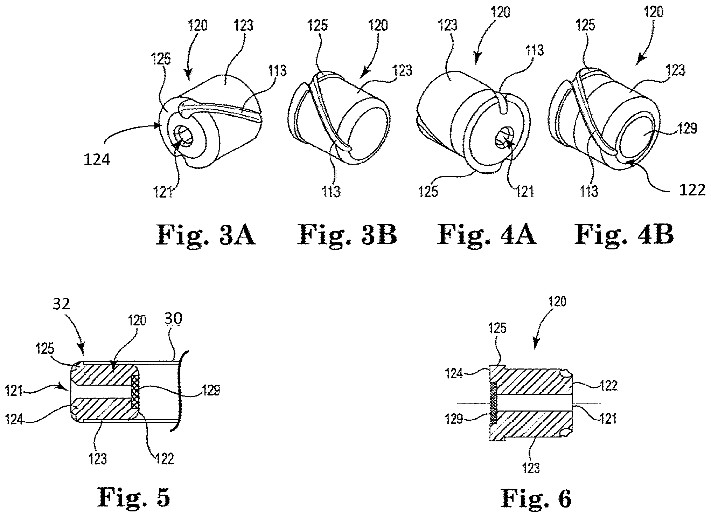

**FIG. 3A** to **FIG. 6** views of an illustrative airflow element **120.** **FIG. 3A** to **FIG. 4B** illustrate opposing curved, or helical air channels **113** rotating about the circumference as a function of a location along the length of the airflow element **120.** The airflow element **120** may include a collar element **125** having a larger diameter than the remaining body **123** of the airflow element **120.**

**FIG. 3A, FIG. 4A, FIG. 5** and **FIG. 6** illustrate a linear piercing channel **121** extending through the length of the airflow element **120.** The linear piercing channel **121** may extend along a central axis of the airflow element **120.** The linear piercing channel **121** may coincide with the longitudinal axis **L_{A}** of the dry powder inhaler **10.** The linear piercing channel **121** may extend the entire lateral length of the airflow element **120** (while the resealable element **129** may occlude a portion of the linear piercing channel **121** length.

**FIG. 4B, FIG. 5** and **FIG. 6** illustrate a resealable element **129** disposed on or within or occluding the linear piercing channel **121.** **FIG. 4B** and **FIG. 5** illustrate a resealable element **129** disposed on the airflow element first lateral face **122** and sealing the linear piercing channel **121.** **FIG. 6** illustrates a resealable element **129** disposed on the airflow element second lateral face **124** and sealing the linear piercing channel **121.** The resealable membrane **129** may form a portion of the first lateral face **122** or second lateral face **124.**

The resealable membrane **129** may define a portion of an upstream boundary of the capsule cavity **26** when the mouthpiece portion **20** is coupled to the distal end portion **30.** The resealable membrane **129** may coincide with the longitudinal axis **L_{A}** of the inhaler **10.**

The airflow element **120** may include two airflow channels **113** extending the longitudinal length of the airflow element **120** and is non-parallel with the longitudinal axis. The airflow channels **113** produce swirling air flow to induce rotation of a capsule contained within the capsule cavity **26.** The airflow element **120** has a longitudinal length defined between a first lateral face **122** and an opposing second lateral face **124.** The airflow channel **113** extends between the first lateral face **122** and the second lateral face **124.**

The first lateral face **122** may define an upstream boundary of the capsule cavity **26** when the mouthpiece portion **20** is coupled to the distal end portion **30.**

**FIG 7** is a schematic diagram of another illustrative dry powder inhaler **10.** The dry powder inhaler body **12** is illustrated with the distal end portion **30** un-coupled from the mouthpiece portion **20** and a capsule **150** within the capsule cavity **26.** This illustrative dry powder inhaler **10** includes a piercing element **37** coupled to the distal end portion **30** and adjacent to the distal end **32.** The piercing element **37** is configured to move between a relaxed position and a piercing position, the piercing element extending through the resealable membrane and into the capsule cavity in the piercing position, the piercing element being spaced apart from the resealable membrane in the relaxed position.

The mouthpiece portion **20** includes a capsule cavity **26** defined within the first mating end **24** and a mouthpiece air channel **25** extending from the mouthpiece end **22** to the capsule cavity **26.** A capsule cavity outlet air channel **23** may provide airflow communication with the mouthpiece air channel **25** to the capsule cavity **26.**

The distal end portion **30** includes an airflow element **120** disposed within the distal end portion **30** and extending a longitudinal length to the capsule cavity **26.** The airflow element **120** includes an airflow channel **113** extending the longitudinal length of the airflow element **120** and is non-parallel with the longitudinal axis **L_{A}**, the airflow channel **113** directs inlet air into the capsule cavity **26.** The airflow element **120** includes a resealable membrane **129** configured to reseal when a piercing element is withdrawn from the resealable membrane **129.**

The distal end portion **30** airflow element **120** may form the upstream boundary of the capsule cavity **26** at the first mating end **24** when the mouthpiece end **20** is coupled to the distal end **30.** The piercing element **36** is configured to move between a relaxed position (**FIG. 7**) and a piercing position (not shown). The piercing needle **37** extends through the linear piercing channel **121** and through the resealable membrane **129** and into the capsule cavity **26** (forming a single aperture in the capsule **150**) in the piercing position. The resealable membrane **129** is configured to reseal when the piercing needle **37** moves from the piercing position to the relaxed position. A biasing element **31** or spring may provide the force to return the piercing element **36** (piercing needle **37**) to the relaxed position. The first mating end **24** is complementary with the second mating end **34** and may form a generally air-tight connection.

During use, inlet air enters the distal lateral face of the airflow element **120** and flows along the length of the airflow element **120** along the airflow channel exiting the opposing lateral face of the airflow element **120** in an airflow current that causes the capsule **150** to rotate within the capsule cavity **26.**

One or more air inlets **28, 38** on the inhaler body may align to provide inlet air to the capsule cavity **28.** The one or more air inlets **28, 38** may extend through a side wall forming the capsule cavity **26.** The air inlets **28, 38** may align with or be in air communication with the air inlets **117** of the receptacle article **100.** The receptacle cavity **26** is configured to mate with the receptacle article **100.** The detachable distal portion **30** may be removed from the mouthpiece portion **20** to expose the receptacle cavity **26** to replace the modular and used or depleted receptacle article **100** with an un-used or full receptacle article **100.**

## Claims

1. A dry powder inhaler (10), comprising:
an inhaler body (12) extending from a mouthpiece end (22) to a distal end (32), the inhaler body comprising a mouthpiece portion (20) removably coupled to a distal end portion (30), the mouthpiece portion extending between the mouthpiece end and a first mating end (24), the distal end portion extending between a second mating end (34) and the distal end (32),
the mouthpiece portion (20) comprising:
a capsule cavity (26) defined within the first mating end (24);
a mouthpiece air channel (25) extending from the mouthpiece end (22) to the capsule cavity (26);
the distal end portion (30) comprising:
an airflow element (120) disposed within the distal end portion and extending a longitudinal length to the capsule cavity (26), the airflow element comprises:
an airflow channel (113) extending the longitudinal length of the airflow element (120) and is non-parallel with the longitudinal axis, the airflow channel directs inlet air into the capsule cavity (26), and
a resealable membrane (129) configured to reseal when a piercing element is withdrawn from the resealable membrane.

2. The dry powder inhaler (10) according to claim 1, wherein the airflow element (120) comprises two airflow channels (113) extending the longitudinal length of the airflow element and is non-parallel with the longitudinal axis, the airflow channels produce swirling air flow to induce rotation of a capsule contained within the capsule cavity (26).

3. The dry powder inhaler (10) according to claim 1 or 2, wherein the airflow element (120) has a longitudinal length defined between a first lateral face (122) and an opposing second lateral face (124), the airflow channel extends between the first lateral face and the second lateral face.

4. The dry powder inhaler (10) according to claim 3, wherein the resealable membrane (129) forms a portion of the first lateral face (122) or second lateral face (124).

5. The dry powder inhaler (10) according to claim 3 or 4, wherein the first lateral face (122) defines an upstream boundary of the capsule cavity (26).

6. The dry powder inhaler (10) according to any preceding claim, wherein the airflow channel (113) defines a curved or arcuate or helical path along the longitudinal length of the airflow element (120).

7. The dry powder inhaler (10) according to any preceding claim, wherein the airflow channel (113) is curved and rotates around a circumference of the airflow element (120) as a function of a location along the airflow element longitudinal length.

8. The dry powder inhaler (10) according to any preceding claim, wherein the airflow channel (113) comprises at least two opposing helical air channels that are symmetrical and each extend along the airflow element (120) longitudinal length.

9. The dry powder inhaler (10) according to any preceding claim, wherein the airflow element (120) comprises a linear piercing channel (121) extending the longitudinal length of the airflow element and the resealable membrane (129) occludes the linear piercing channel.

10. The dry powder inhaler (10) according to any preceding claim, further comprising a piercing element (37) coupled to the distal end portion (30) and adjacent to the distal end (32), the piercing element being configured to move between a relaxed position and a piercing position, the piercing element extending through the resealable membrane (129) and into the capsule cavity (26) in the piercing position, the piercing element being spaced apart from the resealable membrane in the relaxed position.

11. The dry powder inhaler (10) according to any preceding claim, further comprising a receptacle disposed within the capsule cavity (26), the receptacle being a replaceable article (100) of the inhaler, the receptacle is configured to contain a capsule.

12. A dry powder inhaler system comprising:
an inhaler (10) according to any preceding claim; and
a capsule disposed within the capsule cavity (26) of the inhaler.

13. The dry powder inhaler system according to claim 12, wherein the capsule contains solid particles comprising nicotine.

14. The dry powder inhaler system according to claim 12, wherein the capsule contains pharmaceutically active particles comprising nicotine and flavour particles.

## Patentansprüche

1. Trockenpulverinhalator (10), aufweisend:
einen Inhalatorkörper (12), der sich von einem Mundstückende (22) zu einem distalen Ende (32) erstreckt, wobei der Inhalatorkörper einen Mundstückabschnitt (20) aufweist, der mit einem distalen Endabschnitt (30) entfernbar gekoppelt ist, wobei sich der Mundstückabschnitt zwischen dem Mundstückende und einem ersten Paarungsende (24) erstreckt und sich der distale Endabschnitt zwischen einem zweiten Paarungsende (34) und dem distalen Ende (32) erstreckt,
wobei der Mundstückabschnitt (20) aufweist:
einen Kapselhohlraum (26), der innerhalb des ersten Paarungsendes (24) definiert ist;
einen Mundstückluftkanal (25), der sich von dem Mundstückende (22) zu dem Kapselhohlraum (26) erstreckt;
wobei der distale Endabschnitt (30) aufweist:
ein Luftstromelement (120), das innerhalb des distalen Endabschnitts angeordnet ist und sich in Längsrichtung zu dem Kapselhohlraum (26) erstreckt, das Luftstromelement umfassend:
einen sich über die Längslänge des Luftstromelements (120) erstreckenden und nicht parallel zur Längsachse verlaufenden Luftstromkanal (113), der Einlassluft in den Kapselhohlraum (26) leitet, und
eine wiederverschließbare Membran (129), die zum Wiederverschließen ausgelegt ist, wenn ein Durchstechelement aus der wiederverschließbaren Membran herausgezogen wird.

2. Trockenpulverinhalator (10) nach Anspruch 1, wobei das Luftstromelement (120) zwei sich über die Längslänge des Luftstromelements erstreckende Luftstromkanäle (113) aufweist und nicht parallel zur Längsachse verläuft, wobei die Luftstromkanäle einen wirbelnden Luftstrom erzeugen, um die Drehung einer innerhalb der Kapselhöhle (26) enthaltenen Kapsel zu bewirken.

3. Trockenpulverinhalator (10) nach Anspruch 1 oder 2, wobei das Luftstromelement (120) eine Längslänge aufweist, die zwischen einer ersten Seitenfläche (122) und einer gegenüberliegenden zweiten Seitenfläche (124) definiert ist, wobei sich der Luftstromkanal zwischen der ersten Seitenfläche und der zweiten Seitenfläche erstreckt.

4. Trockenpulverinhalator (10) nach Anspruch 3, wobei die wiederverschließbare Membran (129) einen Abschnitt der ersten Seitenfläche (122) oder der zweiten Seitenfläche (124) bildet.

5. Trockenpulverinhalator (10) nach Anspruch 3 oder 4, wobei die erste Seitenfläche (122) eine vorgelagerte Begrenzung des Kapselhohlraums (26) definiert.

6. Trockenpulverinhalator (10) nach einem beliebigen vorhergehenden Anspruch, wobei der Luftstromkanal (113) einen gekrümmten oder bogenförmigen oder schraubenförmigen Weg entlang der Längslänge des Luftstromelements (120) definiert.

7. Trockenpulverinhalator (10) nach einem beliebigen vorhergehenden Anspruch, wobei der Luftstromkanal (113) gekrümmt ist und sich in Abhängigkeit von einer Stelle entlang der Längslänge des Luftstromelements um einen Umfang des Luftstromelements (120) dreht.

8. Trockenpulverinhalator (10) nach einem beliebigen vorhergehenden Anspruch, wobei der Luftstromkanal (113) wenigstens zwei gegenüberliegende symmetrische schraubenförmige Luftkanäle aufweist, die sich jeweils entlang der Längsrichtung des Luftstromelements (120) erstrecken.

9. Trockenpulverinhalator (10) nach einem beliebigen vorhergehenden Anspruch, wobei das Luftstromelement (120) einen linearen Durchstechkanal (121) aufweist, der sich über die Längslänge des Luftstromelements erstreckt, und die wiederverschließbare Membran (129) den linearen Durchstechkanal verschließt.

10. Trockenpulverinhalator (10) nach einem beliebigen vorhergehenden Anspruch, ferner umfassend ein mit dem distalen Endabschnitt (30) gekoppeltes und an das distale Ende (32) angrenzendes Durchstechelement (37), das zur Bewegung zwischen einer entspannten Position und einer Durchstechposition ausgelegt ist, wobei sich das Durchstechelement in der Durchstechposition durch die wiederverschließbare Membran (129) und in den Kapselhohlraum (26) erstreckt, wobei das Durchstechelement in der entspannten Position von der wiederverschließbaren Membran beabstandet ist.

11. Trockenpulverinhalator (10) nach einem beliebigen vorhergehenden Anspruch, ferner aufweisend eine innerhalb des Kapselhohlraums (26) angeordnete Aufnahme, wobei die Aufnahme ein auswechselbarer Artikel (100) des Inhalators ist, wobei die Aufnahme zum Enthalten einer Kapsel ausgelegt ist.

12. Trockenpulverinhalatorsystem, umfassend:
einen Inhalator (10) nach einem beliebigen vorhergehenden Anspruch; und
eine innerhalb des Kapselhohlraums (26) des Inhalators angeordnete Kapsel.

13. Trockenpulverinhalatorsystem nach Anspruch 12, wobei die Kapsel feste Partikel enthält, die Nikotin umfassen.

14. Trockenpulverinhalatorsystem nach Anspruch 12, wobei die Kapsel pharmazeutisch aktive Partikel enthält, die Nikotin und Geschmackspartikel umfassen.

## Revendications

1. Inhalateur à poudre sèche (10) comprenant :
un corps d'inhalateur (12) s'étendant d'une extrémité d'embout buccal (22) jusqu'à une extrémité distale (32), le corps d'inhalateur comprenant une partie d'embout buccal (20) couplée de manière amovible à une partie d'extrémité distale (30), la partie d'embout buccal s'étendant entre l'extrémité d'embout buccal et une première extrémité d'accouplement (24), la partie d'extrémité distale s'étendant entre une deuxième extrémité d'accouplement (34) et l'extrémité distale (32),
la partie d'embout buccal (20) comprenant :
une cavité de capsule (26) définie à l'intérieur la première extrémité d'accouplement (24) ;
un conduit d'air d'embout buccal (25) s'étendant de l'embout buccal (22) vers la cavité de capsule (26) ;
la partie d'extrémité distale (30) comprenant :
un élément d'écoulement d'air (120) disposé à l'intérieur de la partie d'extrémité distale et s'étendant sur une longueur longitudinale jusqu'à la cavité de capsule (26), l'élément d'écoulement d'air comprend :
un conduit d'écoulement d'air (113) s'étendant sur la longueur longitudinale de l'élément d'écoulement d'air (120) et n'est pas parallèle à l'axe longitudinal, le conduit d'écoulement d'air dirige l'air d'entrée dans la cavité de capsule (26), et
une membrane refermable (129) configurée pour se refermer lorsqu'un élément de perçage est retiré de la membrane refermable.

2. Inhalateur à poudre sèche (10) selon la revendication 1, dans lequel l'élément d'écoulement d'air (120) comprend deux conduits d'écoulement d'air (113) s'étendant sur la longueur longitudinale de l'élément d'écoulement d'air et n'est pas parallèle à l'axe longitudinal, les conduits d'écoulement d'air produisent un écoulement d'air tourbillonnant afin de provoquer la rotation d'une capsule contenue à l'intérieur de la cavité de capsule (26).

3. Inhalateur à poudre sèche (10) selon la revendication 1 ou 2, dans lequel l'élément d'écoulement d'air (120) a une longueur longitudinale définie entre une première face latérale (122) et une deuxième face latérale opposée (124), le conduit d'écoulement d'air s'étend entre la première face latérale et la deuxième face latérale.

4. Inhalateur à poudre sèche (10) selon la revendication 3, dans lequel la membrane refermable (129) forme une partie de la première face latérale (122) ou de la deuxième face latérale (124).

5. Inhalateur à poudre sèche (10) selon la revendication 3 ou 4, dans lequel la première face latérale (122) définit une limite amont de la cavité de capsule (26).

6. Inhalateur à poudre sèche (10) selon l'une quelconque des revendications précédentes, dans lequel le conduit d'écoulement d'air (113) définit un trajet incurvé ou arqué ou hélicoïdal le long de la longueur longitudinale de l'élément d'écoulement d'air (120) .

7. Inhalateur à poudre sèche (10) selon l'une quelconque des revendications précédentes, dans lequel le conduit d'écoulement d'air (113) est incurvé et tourne autour d'une circonférence de l'élément d'écoulement d'air (120) en fonction d'un emplacement le long de la longueur longitudinale de l'élément d'écoulement d'air.

8. Inhalateur à poudre sèche (10) selon l'une quelconque des revendications précédentes, dans lequel le conduit d'écoulement d'air (113) comprend au moins deux conduits d'air hélicoïdaux opposés qui sont symétriques et s'étendent chacun le long de la longueur longitudinale de l'élément d'écoulement d'air (120).

9. Inhalateur à poudre sèche (10) selon l'une quelconque des revendications précédentes, dans lequel l'élément d'écoulement d'air (120) comprend un conduit de perçage linéaire (121) s'étendant sur la longueur longitudinale de l'élément d'écoulement d'air et la membrane refermable (129) obture le conduit de perçage linéaire.

10. Inhalateur à poudre sèche (10) selon l'une quelconque des revendications précédentes, comprenant en outre un élément de perçage (37) couplé à la partie d'extrémité distale (30) et adjacent à l'extrémité distale (32), l'élément de perçage étant configuré pour se déplacer entre une position relâchée et une position de perçage, l'élément de perçage s'étendant à travers la membrane refermable (129) et dans la cavité de capsule (26) dans la position de perçage, l'élément de perçage étant espacé de la membrane refermable dans la position relâchée.

11. Inhalateur à poudre sèche (10) selon l'une quelconque des revendications précédentes, comprenant en outre un réceptacle disposé à l'intérieur de la cavité de capsule (26), le réceptacle étant un article remplaçable (100) de l'inhalateur, le réceptacle est configuré pour contenir une capsule.

12. Système inhalateur à poudre sèche comprenant :
un inhalateur (10) selon l'une quelconque revendication précédente ; et
une capsule disposée à l'intérieur de la cavité de capsule (26) de l'inhalateur.

13. Système inhalateur à poudre sèche selon la revendication 12, dans lequel la capsule contient des particules solides comprenant de la nicotine.

14. Système inhalateur à poudre sèche selon la revendication 12, dans lequel la capsule contient des particules pharmaceutiquement actives comprenant de la nicotine et des particules d'arôme.
